# EUROPEAN PATENT SPECIFICATION

(11) **EP 2 589 492 B1**
(45) Date of publication and mention of the grant of the patent: **16.12.2015**
(21) Application number: 11812178.9
(22) Date of filing: 15.06.2011
(51) Int. Cl.: C09J 7/04, C09J 7/02, A61L 15/58, A61F 13/02

(54) **SUPPORTING FILM FOR TAPE MATERIAL AND TAPE MATERIAL**
TRÄGERFOLIE FÜR EIN BANDMATERIAL UND BANDMATERIAL
FILM DE SUPPORT POUR MATÉRIAU EN RUBAN, ET MATÉRIAU EN RUBAN

(30) Priority: 29.07.2010 JP 2010171209
(43) Date of publication of application: 08.05.2013
(73) Proprietor: Hisamitsu Pharmaceutical Co., Inc., Tosu-shi, Saga 841-0017 (JP); Toppan Printing Co., Ltd., Tokyo 110-8560 (JP); Maruto Sangyo Co., Ltd., Ogori-shi, Fukuoka 838-0112 (JP)
(72) Inventor: TAKADA Yasunori, Tsukuba-shi Ibaraki 305-0856 (JP); SHIMA Takito, Tsukuba-shi Ibaraki 305-0856 (JP); TATEISHI Tetsurou, Tosu-shi Saga 841-0017 (JP); NISHIHARA Tsuguki, Tokyo 110-8560 (JP); YOSHIDA Chiaki, Tokyo 110-8560 (JP); MATSUSHIMA Atsushi, Tokyo 110-8560 (JP); TAKAMIYA Tsuyoshi, Ogori-shi Fukuoka 838-0112 (JP)
(74) Representative: Prüfer & Partner GbR European Patent Attorneys
(86) International application number: PCT/JP2011/063656
(87) International publication number: WO 2012/014585

(56) References cited:
- EP-A2- 0 164 594
- GB-A- 2 452 086
- JP-A- 9 262 249
- JP-A- 2003 136 645
- US-A- 3 549 452

## Description

### BACKGROUND OF THE INVENTION

### Field of the Invention

The present invention relates to a support film for tape, and particularly a support film for tape having barrier properties, and a tape using the same.

### Description of Related Art

A tape on which an adhesive layer is formed on one surface of a sheet-shaped or film-shaped support has been widely used for various purposes such as for medical use or industrial use. In addition to adhesive materials, in some cases, a plasticizer and the like are combined with the adhesive layer of the tape. Since there is a concern of a negative effect due to adsorption of the plasticizer depending on materials of a support, it is preferable that at least a surface of the support which comes into contact with the adhesive layer has barrier properties.

A method of producing a laminated adhesive product with a barrier layer is for example described in US 3549452 A. This tape is produced by applying a liquid polyisocyanate to the plastic layer, followed by an aqueous solution of polyvinyl alcohol resin and applying an adhesive layer to the dried surface. The laminate is made of vinyl resin such as a polyvinyl chloride resin.

EP 0164594 A2 shows a heat-laminating composite film comprising a saponified product of an ethylene-vinyl acetate copolymer, a reactive polyurethane containing isocyanate groups and a vinyl chloride copolymer.

GB 2452086 A discloses gas barrier materials containing PVA and/or EVOH with clay as intermediate layer between two films for packaging food stuff and pharmaceuticals.

A further gas barrier film disclosed in Patent Document 1 (Japanese Unexamined Patent Application, First Publication No. 2003-136645) has been used as a film material having barrier properties. In this gas barrier film, a barrier coating film is formed by applying a barrier coating material which is obtained by mixing montmorillonite, which is a layered inorganic compound, and a water-soluble polymer compound on one surface of a plastic base film.

A tape is used in a severe environment to come in contact with or be dipped into liquid in a state of being attached to an object, in some cases. However, since a gas barrier film disclosed in Patent Document 1 is not initially obtained by assuming uses under such environments, an adhesive property of a support and a barrier coating layer is regarded as being insufficient. Accordingly, when trying to apply the gas barrier film disclosed in Patent Document 1 to a tape as it is, there are problems in that peeling is generated between the support and the barrier coating layer, and the gas barrier film does not sufficiently resist a severe usage environment.

### SUMMARY OF THE INVENTION

The present invention has been made to address the aforementioned problems and aims at providing a support film for tape and a tape which can be used while maintaining an excellent barrier property even under a severe environment.

A first aspect of the present invention provides a support film for tape which is used for a tape, including a film-shaped support formed of polyurethane; and a barrier layer which includes polyvinyl alcohol and a layered inorganic compound, and which is formed on one surface of the support, in which a degree of saponification of the polyvinyl alcohol is equal to or more than 70 percent and equal to or less than 95.5%, and wherein the film-shaped support can be elongated by a predetermined maximum elongation rate increasing equal to or more than 10% dimensionally.

In the support film for tape of the present invention, it is desirable that the layered inorganic compound be montmorillonite.

In addition, a tape of a second aspect of the present invention includes: the support film for tape of the present invention; and an adhesive layer which is formed on the barrier layer opposite to the support.

A support film for tape and a tape of the present invention having an excellent barrier property under a severe environment can be maintained during use.

### BRIEF DESCRIPTION OF THE DRAWINGS

FIG. 1 is a cross-sectional view of a tape of an embodiment of the present invention in a thickness direction.
FIG. 2 is a view showing a procedure of an experiment for checking for a suitable range of a percentage content of montmorillonite in a barrier layer.
FIG. 3 is a view showing a procedure of the same experiment.
FIG. 4 is a view showing a procedure of the same experiment.
FIG. 5 is a view showing a procedure of the same experiment.
FIG. 6 is an optical micrograph of a barrier layer after performing an elongation operation with an elongation rate of 20% with respect to an evaluated piece with a percentage content of montmorillonite in a barrier layer of 10 wt%.
FIG. 7 is an optical micrograph of a barrier layer after performing an elongation operation with an elongation rate of 20% with respect to an evaluated piece with a percentage content of montmorillonite in a barrier layer of 18 wt%.
FIG. 8 is an optical micrograph of a barrier layer after performing an elongation operation with an elongation rate of 20% with respect to an evaluated piece with a percentage content of montmorillonite in a barrier layer of 25 wt%.
FIG. 9 is an optical micrograph of a barrier layer after performing an elongation operation with an elongation rate of 20% with respect to an evaluated piece with a percentage content of montmorillonite in a barrier layer of 30 wt%.
FIG. 10 is a graph showing a relationship between a percentage content of montmorillonite and a modulus value of a support film for tape.
FIG. 11 is a view showing a procedure of an experiment for checking for a relationship between a degree of saponification of a water-soluble polymer compound and an adhesiveness of a support-barrier layer.
FIG. 12 is a view showing a procedure of the same experiment.
FIG. 13 is a view showing a procedure of the same experiment.
FIG. 14 is a view showing a procedure of the same experiment.
FIG. 15 is a view showing a procedure of the same experiment.

### DETAILED DESCRIPTION OF THE INVENTION

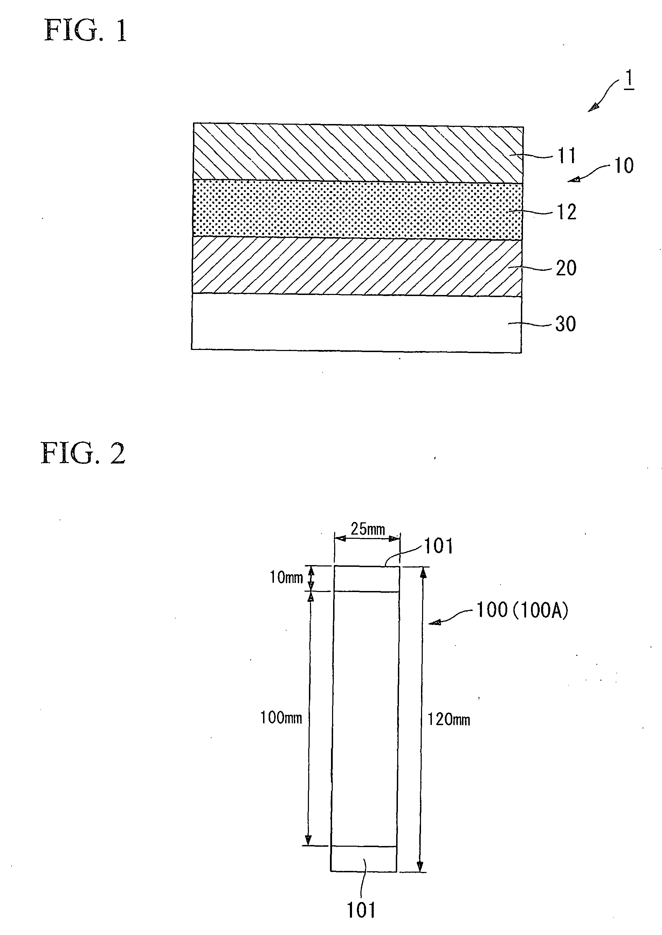

Hereinafter, a tape of an embodiment of the present invention will be described referring to FIGS. 1 to 15. The tape of the embodiment is configured to include a support film for tape (hereinafter, simply referred to as "support film") of the present invention, and the tape can be used as an adhesive tape or the like in various fields such as for industrial use, packaging, protecting, labeling, masking, hygienic materials such as a diaper, medical use such as an adhesive patch or percutaneously administered medicine, make-up, and household use.

In addition, the following are exemplified as representative detailed examples. An adhesive tape for locking a packaging material is used as a packaging. As the adhesive tape is capable of being elongated, even in a case of applying a force due to shipping or the like in a state of being packaged, the tape is not cut or the packaging material is not damaged. In addition, since it is possible to maintain excellent barrier properties in a state of being elongated, an exterior portion is not contaminated when shipping or the like. For medical use, in a case of attaching to a human body or an animal, it is possible to respond to according to elongation associated with movement of a human body or the like, and unexpected peel-off is hardly generated. In addition, since it is possible to maintain excellent barrier properties in a state of being elongated, it is possible to obtain excellent performances for medical use without leakage of an active ingredient such as a plasticizer or the like, to the external portion.

FIG 1 is a cross-sectional view of a tape 1 of the embodiment in a thickness direction. The tape 1 includes a support film 10, an adhesive layer 20 formed on one surface of the support film 10, and a peel-off member 30 which covers the adhesive layer.

The support film 10 includes a support 11 which includes polyurethane and is formed in a film shape, and a barrier layer 12 which is formed on one surface of the support 11.

The support 11 has flexibility and can be elongated by a predetermined maximum elongation rate increasing equal to or more than 10 percent (%) dimensionally. A detailed value of the maximum elongation rate may be suitably set based on the purpose of the tape 1. In the embodiment, the polyurethane which forms the support 11 is not particularly limited, polyurethane used in a polyurethane film of the related art can be used, and the polyurethane can be suitably selected depending on the purpose. For example, polyether-based polyurethane, polyester-based polyurethane, polycarbonate-based polyurethane or the like may be used. To provide necessary water resistance, polyether-based polyurethane or polycarbonate-based polyurethane is preferable.

In addition, it is not particularly limited to a type of isocyanate forming a urethane bond, a yellowing type, or a non-yellowing type, and it is suitably selected according to the purpose, storing period or method in usage, types of used plasticizer and the like.

A thickness of the support 11 is 10 micrometers (µm) to 200 µm, and is preferably equal to or more than 15 µm and equal to or less than 100 µm. When the thickness is less than 10 µm, it is difficult to handle as it is too thin, and when the thickness is more than 200 µm, flexibility is reduced such that original flexibility is not sufficiently exhibited.

The support 11 can include a film called a release film having a peel-off property. When the thickness of the support 11 is thin, since the support is elongated in a step of applying the barrier layer 12, if manufacturing in a state where the release film and the support (for example, polyurethane as the support) are laminated, it is possible to easily process while suppressing the elongation. In addition, since rigidity of the tape is reinforced by the release film after processing the support 11 on the tape, handleability of the tape is improved. The release film can be adhered to the tape to an object and peeled from the support, such that the support 11 after the peel-off exhibits original flexibility.

The material of the release film is not particularly limited; however, generally, a material which can be peeled off without performing elongation or contraction, such as a silicon-treated PET film, a polyolefin film having an excellent peel-off property, an aggregate such as paper or polyethylene, or the like can be used.

The barrier layer 12 is formed to include montmorillonite, which is a layered inorganic compound, and polyvinyl alcohol (PVA), which is a water-soluble polymer compound.

Mineralogically, the montmorillonite is a dioctahedral type water-bearing layered silicate mineral and is ideally expressed by the following equation.

(Al_{2-y}Mg_{y})Si₄O₁₀(OH)₂ · (M⁺,M_{1/2}²⁺)y · nH₂O

Herein, y = 0.2 to 0.6, M: exchangeable cation such as Na, K, Ca, Mg, or H, n: amount of interlayer water.

A crystal structure of montmorillonite forms a layered structure which includes three layers formed of two tetrahedral sheets and one octahedral sheet as a base. A cation of the tetrahedral sheets is only Si, and a cation, Al, of the octahedral sheet is substituted for a part of Mg. Accordingly, a unit crystal layer takes on a negative electric charge, and cations such as Na⁺, K⁺, Ca²⁺, Mg²⁺, H⁺, and the like enter and compensate between crystal layers so as to balance with the negative electric charge. In the present invention, types of the cation can be used with no particular limitations.

The barrier layer 12 can be formed by applying a barrier coating material obtained by adding and adjusting lower alcohol with a gravure coating method or a roll coating method, after the montmorillonite is added to and dispersed in a water solution obtained by melting PVA in water. If necessary, an anchor coating layer may be formed on the support 11 and the barrier layer 12 may be formed on the anchor coating layer. In the same manner, the barrier layer 12 may be formed after being subjected to a surface treatment on the support 11. As the surface treatment, a corona discharge treatment or a plasma discharge treatment is preferable. From the above, the corona discharge treatment is more preferable from the viewpoint of general versatility or handleability.

A percentage content of the montmorillonite of the barrier layer 12 is in a range of equal to or more than 2 weight percent (wt%) and equal to or less than 22 wt%. A detailed description will be described later; however, if the percentage content is less than 2 wt%, it is difficult to secure a sufficient barrier property. On the other hand, if the percentage content exceeds 22 wt%, an effect caused by the montmorillonite on the physical property of the barrier layer 12 becomes too much, and as a result, sufficient responding to shape change of the support due to the elongation cannot be performed, and cracks or the like are easily generated.

In addition, when the tape 1 is used in an environment to be placed in water for long time in a state of being attached to an object, for example, if adhesiveness of the support 11 and the barrier layer 12 is not sufficient, the support 11 is peeled off from the barrier layer 12 and separated from the adhesive layer 20, in some cases. The PVA is a polymer compound which is obtained by saponification of polyvinyl acetate (alkaline hydrolysis treatment) and includes a hydroxyl group; however, in the support film 10 of the embodiment, in order to maintain excellent adhesiveness of the barrier layer 12 and the support 11 to prevent the situation described above, a degree of saponification of PVA is in a range equal to or more than 70% and equal to or less than 95.5%. A detailed description thereof will be also described later; however, if the degree of saponification exceeds 95.5%, the adhesiveness with the support 11 is degraded, and if the degree of saponification is less than 70%, the barrier layer 12 becomes easily melted in water, and as a result, water resistance of the support film 10 and the tape 1 is degraded. Accordingly, when used for industrials, packaging, or protecting, it is assumed to be affected by rain outside, and when used for hygienic materials such as a diaper, medical use such as an adhesive patch or percutaneously administered medicine, make-up, or household use, it is assumed to be affected by sweat or water when using water in daily life.

The adhesive layer 20 is configured by mixing plasticizer with a base material having an adhesive property and is formed by applying or the like on the barrier layer 12 and a surface opposite to the support 11.

An adhesive used in the adhesive layer 20 is not particularly limited, and a rubber-based polymer such as natural rubber, synthetic isoprene rubber, reclaimed rubber, styrene-butadiene rubber (SBR), styrene-isoprene-styrene block copolymer (SIS), styrene-butadiene-styrene block copolymer (SBS), polyisobutylene, SEBS, SEPS, or the like, acrylic polymers such as a copolymer of (meth) acrylic acid ester containing (meth) acrylic acid ester as a main monomer, silicon-based polymers such as silicon rubber, silicon resin, dimethyl siloxane, diphenyl siloxane, and the like, and various polyvinyl ether-based, polyvinyl ester-based, EVA-based, polyester-based materials can be used. The plasticizer is not particularly limited, and various plasticizers such as petroleum-based oil (paraffinic process oil, naphthenic process oil, or aromatic process oil), dibasic acid esters (dibutyl phthalate, or dioctyl phthalate), liquid rubbers (polybutene, liquid isoprene, or liquid polyisobutylene), vegetable-based oils (castor oil or tall oil) liquid fatty acid esters (isopropyl myristate, hexyl laurate, diethyl sebacate, or diisopropyl sebacate), triacetin, sorbitan fatty acid esters, sucrose fatty acid esters, glycerin fatty acid esters, and surfactants can be used.

Further, for improving adhesiveness, various tackifiers can be mixed. For example, rosin resins such as rosin, modified rosin, or rosin ester, terpene resins such as terpene resin, aromatic modified terpene resin, hydrogenated terpene resin, or terpene phenol resin, petroleum resin such as aliphatic petroleum resins, aromatic petroleum resins, copolymer petroleum resin, hydrogenated petroleum resin, or DCPD-based petroleum resin, styrene resins, styrene resins substituted, xylene resin, phenol resin, chroman-indene resin or the like can be used.

In addition, depending on the purpose of the tape, antioxidants, fillers, cross-linking agents, ultraviolet absorbers, colorants, flame retardants, conductive agents, foaming agents, or the like may be added.

In the tape 1 of the embodiment, by suitably setting the percentage content of montmorillonite of the barrier layer 12 in the range described above while considering the types of plasticizers, transition of the plasticizer to the support 11 from the barrier layer 12 is suitably suppressed.

In general, polyurethane configuring the support 11 is easily adsorbed onto the plasticizer, and in this case, transformation or the like of the support 11 due to the transition of the plasticizer to the support 11 becomes a problem; however, in the tape 1, not only at the time of non-elongation of the support 11, but even at the time of elongation with the elongation rate of 20% (which indicates an increase of length by 20% after the elongation), the barrier property of the barrier layer 12 is suitably maintained. As a result, not only at the time of storage before the use, but also at the time of being used by being attached to an object, it is possible to suitably prevent the problem due to the plasticizer. In addition, the percentage content of the montmorillonite can be easily set in detail by a preliminary experiment or the like using the plasticizer to be used. A relationship between the barrier property with respect to a part of the plasticizer and the percentage content of montmorillonite will be described later.

The peel-off member 30 is a member to protect an adhesive surface of the adhesive layer 20 until the adhesion to an object, and various types of well-known release paper can be suitably used. In addition, when the tape 1 is rolled up on a core, the peel-off member 30 may not be prepared.

Next, a test and a result thereof which is performed for evaluating a suitable range of the percentage content of the montmorillonite (hereinafter, referred to as "MN", in some cases) of the barrier layer 12 and a suitable range of the degree of saponification of the PVA will be described.

### (Experiment 1 Evaluation of Relationship between Barrier Property and Percentage Content of MN at the Time of Elongation: Evaluation with Swelling of Support as Index)

### (1-1 Preparation of Sample)

As a support, a material prepared by polyether-based polyurethane having a thickness of 20 µm was used. A barrier layer was formed by uniformly applying 1.0 g/m² of a barrier coating material which was obtained by mixing MN and PVA (with a degree of saponification of 80%) on one surface of the support. By setting this as a basic configuration, 8 stages of the percentage content of MN of the barrier layer were 1 wt%, 2 wt%, 10 wt%, 18 wt%, 22 wt%, 25 wt%, 30 wt%, and 37 wt%, and 8 types of samples of support films were prepared.

### (1-2 Experiment Procedure)

The prepared 8 types of samples 100 were cut to be a size of 25 millimeters (mm) × 120 mm as shown in FIG. 2, and in order to perform easy operation with a tensile tester, a sheet 101 prepared by polyethylene terephthalate (PET) having a thickness of 50 µm was attached to both surfaces of both ends in a longitudinal direction with double-sided tape and an evaluation piece 100A was prepared. A dimension of the evaluation piece 100A of the sheet 101 in the longitudinal direction was 10 mm, and in each evaluation piece 100A, a length of a portion which was not covered with the sheet 101 in the longitudinal direction was 100 mm.

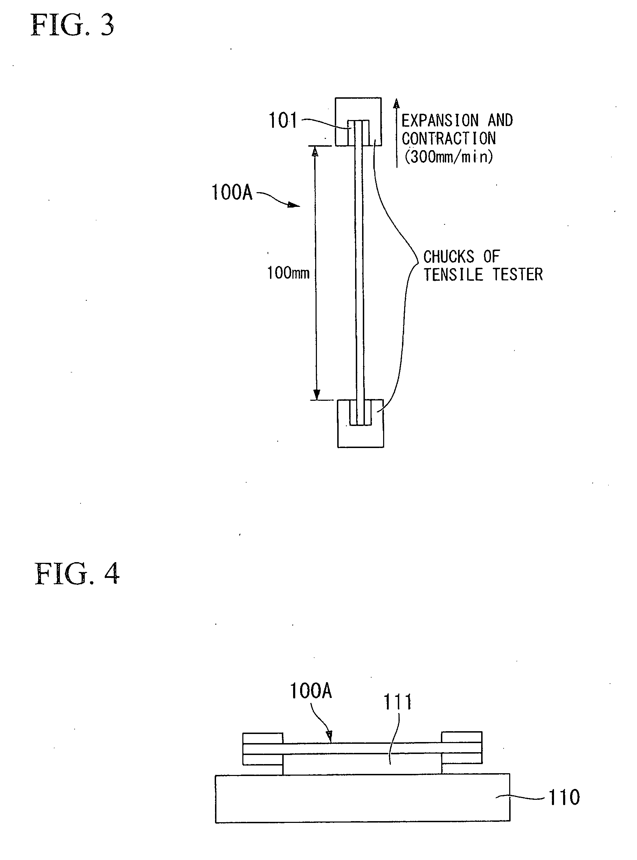

Both ends of the evaluation piece 100A reinforced by the sheet 101 were fixed to the chuck unit of the tensile tester, and as shown in FIG. 3, the portion not covered with the sheet 101 was elongated to reach a predetermined elongation rate with an elongation speed of 300 mm per minute (mm/min). Five stages of elongation rate were 0%, 5%, 10%, 20%, and 30%.

After completing the elongation operation, the evaluation piece 100A was taken off from the tensile tester, and as shown in FIG. 4, each evaluation piece 100A was fixed onto a black acrylic plate 110 obtained by attaching a PET sheet 111 obtained by applying silicon on the surface thereof, with a barrier layer to be on the upper side. At that time, the preparation was performed so that as little air as possible entered between the evaluation piece 100A and the PET sheet 111.

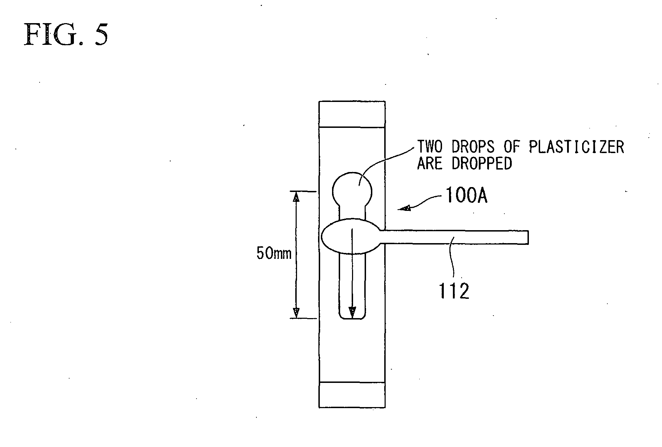

After attaching to the acrylic plate 110, as shown in FIG. 5, two drops (about 0.08 grams) of plasticizers were put on each evaluation piece 100 by a dropper, and the evaluation piece was expanded to have a length of 50 mm by using a cotton swab 112. As plasticizers, four types of isopropyl myristate (IPM), triacetin (TA), glyceryl monoisostearate (MGIS), and sorbitan monooleate (SMO) were used. After being left for 30 minutes at room temperature, the plasticizers were wiped off and a degree of swelling of the support was visually evaluated. As an index, wrinkles of the support member generated due to the swelling were used (two stages of wrinkles due to swelling were not recognized: Good, and wrinkles due to swelling were recognized: Bad).

The IPM, the TA, and the SMO were reviewed using the evaluation pieces 100A having a percentage content of MN of 1 wt%, 10 wt%, 18 wt%, 22 wt%, 25 wt%, 30 wt%, and 37 wt%, and MGIS was reviewed using the evaluation pieces 100A having a percentage content of MN of 2 wt%, 10 wt%, and 22 wt%.

### (1-3 Result)

The result is shown in Table 1. When the percentage content of MN was equal to or less than 22 wt% with the IPM, TA, and MGIS, the swelling of the support with all elongation rates was not recognized, and transition of the plasticizers was suppressed. On the other hand, with the SMO, the swelling was recognized on the support regardless of the percentage content of MN and the elongation rates, and it was considered that the SMO was not preferable as the plasticizer to be used for a film material of the present invention, in some cases. Solubility parameters (SP value based on Fedors method) of each plasticizer used in the experiment were 8.5 for IPM, 10.2 for TA, 10.76 for MGIS, and 11.76 for SMO, and it was assumed that the plasticizer having a low SP value tended to be preferable.

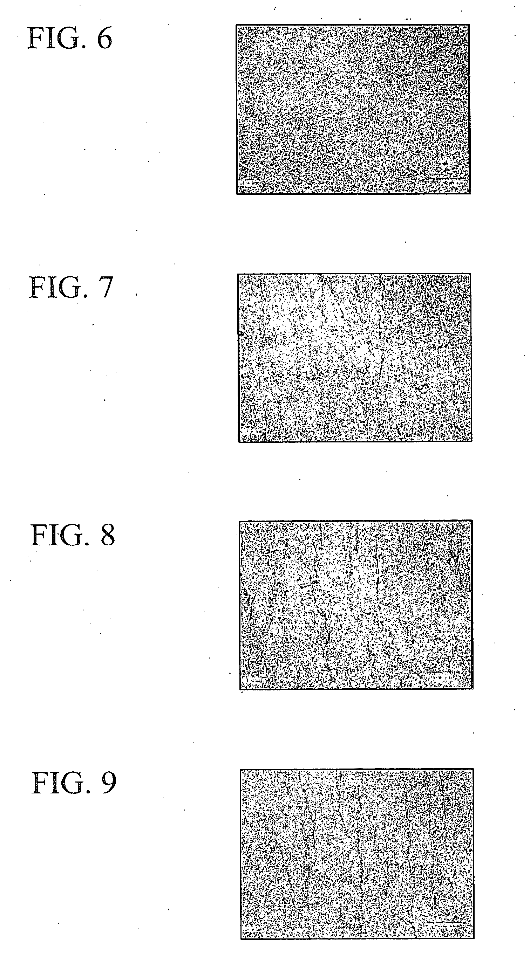

FIGS. 6 to 9 are optical micrographs of the support film after performing the elongation operations with an elongation rate of 20% with respect to the evaluated pieces with a percentage content of MN of 10 wt%, 18 wt%, 25 wt%, and 30 wt%. In a case of 10 wt% and 18 wt% of the MN, significant changes on the external portion were not recognized; however, in a case of 25 wt% and 30 wt% of the MN, wrinkles due to the swelling were recognized.

**[Table 1]**

| Sample configuration | | | Evaluation | | | | | | | | | | | | | | | | | | | |
|---|---|---|---|---|---|---|---|---|---|---|---|---|---|---|---|---|---|---|---|---|---|---|
| Support member | Barrier layer formula | | IPM | | | | | TA | | | | | MGIS | | | | | SMO | | | | |
| | Degree of saponificati on of PVA | Amount of MN | Elongation rate (%) | | | | | Elongation rate (%) | | | | | Elongation rate (%) | | | | | Elongation rate (%) | | | | |
| | % | wt% | 0 | 5 | 10 | 20 | 30 | 0 | 5 | 10 | 20 | 30 | 0 | 5 | 10 | 20 | 10 | 0 | 5 | 10 | 20 | 30 |
| | | 1 | Good | Good | Good | Good | Good | Good | Good | Good | Good | Good | None | None | None | None | None | Bad | Bad | Bad | Bad | Bad |
| | | 2 | None | None | None | None | None | None | None | None | None | None | Good | Good | Good | Good | Good | None | None | None | None | None |
| | | 10 | Good | Good | Good | Good | Good | Good | Good | Good | Good | Good | Good | Good | Good | Good | Good | Sad | Bad | Bad | Bad | Bad |
| Ethers 20 µm | 80 | 18 | Good | Good | Good | Good | Good | Cood | Good | Good | Good | Good | None | None | None | None | None | Bad | Bad | Bad | Bad | Bad |
| | | 22 | Good | Good | Good | Good | Good | Good | Good | Good | Good | Good | Good | Good | Good | Good | Good | Bad | Bad | Bad | Bad | Bad |
| | | 25 | Good | Good | Bad | Bad | Bad | Good | Bad | Bad | Bad | Bad | None | None | None | None | None | Bad | Bad | Bad | Bad | Bad |
| | | 30 | Good | Bad | Bad | Bad | Bad | Good | Bad | Bad | Bad | Bad | None | None | None | None | None | Bad | Bad | Bad | Bad | Bad |
| | | 37 | Bad | Bad | Bad | Bad | Bad | Bad | Bad | Bad | Bad | Bad | None | None | None | None | None | Bad | Bad | Bad | Bad | Bad |

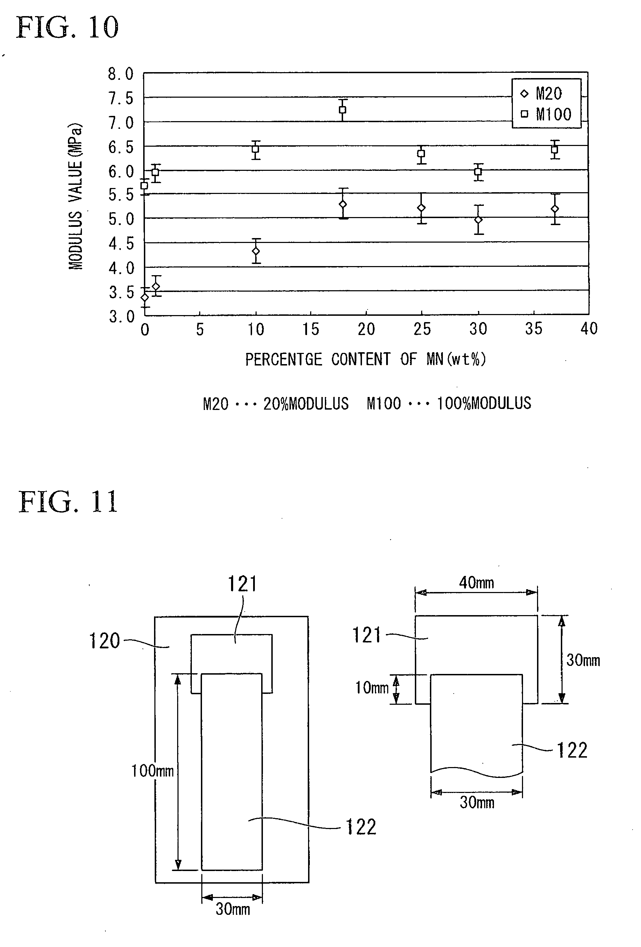

In addition, in each sample, when evaluating modulus after the elongation operation based on a test method of a polyurethane-based thermoplastic elastomer (JIS K 7311), as shown in FIG. 10, the modulus was equal to or less than 8 Megapascals (MPa) for all samples, and excellent flexibility was shown. Accordingly, it was determined that the barrier layer did not negatively affect the flexibility of the support film.

### (Experiment 2 Evaluation of Relationship between Barrier Property and Percentage Content of MN: Evaluation Using Adhesive Layer Containing Plasticizer)

### (2-1 Preparation of Sample)

The same material as Experiment 1 was used as a support, and a barrier layer was formed by uniformly applying 1.0 g/m² of a barrier coating material which was obtained by mixing MN and PVA (with a degree of saponification of 80%) on one surface of the support. Nine stages of the pe rcentage content of MN of the barrier layer were 1 wt%, 2 wt%, 4 wt%, 10 wt%, 18 wt%, 22 wt%, 25 wt%, 30 wt%, and 37 wt%, and 9 types of samples of support films were prepared.

In addition, an adhesive layer (applied amount of adhesive layer: 100 g/m²) including a base material and a plasticizer was formed on the barrier layer. Two types of rubber base material and an acrylic base material were used as the base material, and a total of 5 types of adhesive layer materials were prepared by combining each base material with a plurality of types of plasticizers. The adhesive layer was formed on each sample using each adhesive layer material, and samples of tapes were prepared by covering the adhesive layer with a peel-off member. The combinations of the base material and the plasticizer for each adhesive layer material were as follows (% of the plasticizer indicates the percentage content). Rubber base material (IPM 20%, MGIS 10%, SMO 10%, and SMO 20%) And the acrylic base material (IPM 20%, TA 10%, MGIS 10%, and SMO10%)

### (2-2 Experiment Procedure)

### a. Stability Test for Non-Elongation Time

A tape sample obtained by cutting to be 10 square centimeters was stored at 60°C for 1 week without performing an elongation operation.

### b. Stability Test for Elongation Time

A tape sample obtained by cutting to have a width of 30 mm and a length of 50 mm was stored at 60°C for three days after removing the peel-off member and performing the elongation operation with an elongation rate of 20% in a length direction once.

In all cases, in each tape sample after storing, in the same manner as Experiment 1, the barrier property was evaluated by the generation of wrinkles of the support. The evaluation of Experiment 2 was set as three stages. Wrinkles due to the swelling were not recognized: Excellent, slight wrinkles due to the swelling were recognized, but did not affect the quality: Good, and wrinkles due to the swelling were recognized and the support cannot be used: Bad.

### (2-3 Experiment Results)

The result is shown in Table 2. It is shown that when the percentage content of the MN is 2 wt%, 10 wt%, and 18 wt%, in any of the non-elongation time and the 20% elongation time, wrinkles were not recognized in the support and the barrier property was excellently maintained.

In addition, for the SMO, which was considered to be not preferable some times in Experiment 1, it was determined that the transition of the plasticizers to the support can be sufficiently suppressed by suitably setting the percentage content of MN of the barrier layer or the percentage content of the plasticizers of the adhesive layer.

**[Table 2]**

| Amount of MN (wt%) | Rubber based material | | | | | | | | Acrylic base material | | | | | | | |
|---|---|---|---|---|---|---|---|---|---|---|---|---|---|---|---|---|
| | IPM 20% | | MG1S 10% | | SMO 10% | | SMO 20% | | IPM 20% | | TA 10% | | MGIS 10% | | SMO 10% | |
| | Non elongation | Elongation rate 20% | Non elongation | Elongation rate 20% | Non elongation | Elongation rate 20% | Non elongation | Elongation rate 20% | Non elongation | Elongation rate 20% | Non elongation | Elongation rate 20% | Non elongation | Elongation rate 20% | Non elongation | Elongation rate 20% |
| 1 | Bad | Bad | Bad | Bad | Bad | Bad | Bad | Bad | Bad | Excellent | Bad | Bad | Bad | Bad | Bad | Bad |
| 2 | Excellent | Excellent | Bad | Bad | Bad | Bad | None | None | Excellent | Good | None | None | Bad | Bad | Bad | Bad |
| 4 | None | None | Good | Good | Bad | Bad | None | None | Excellent | Excellent | None | None | Good | Good | Bad | Bad |
| 10 | Excellent | Excellent | Excellent | Excellent | Good | Good | Bad | Bad | Excellent | Excellent | Bad | Bad | Excellent | Good | Good | Excellent |
| 18 | Excellent | Excellent | Excellent | Excellent | Excellent | Excellent | Bad | Bad | Excellent | Excellent | Good | Good | Excellent | Excellent | Excellent | Excellent |
| 22 | Excellent | Excellent | Excellent | Excellent | Excellent | Excellent | None | None | Excellent | Excellent | Excellent | Excellent | Excellent | Excellent | Excellent | Excellent |
| 25 | Bad | Bad | None | None | None | None | Bad | Bad | None | Bad | None | Good | None | None | None | Bad |
| 30 | Bad | Bad | None | None | None | None | Bad | Bad | Excellent | Bad | Excellent | Good | None | None | Excellent | Bad |
| 37 | Bad | Bad | None | None | None | None | Bad | Bad | Good | Bad | Excellent | Good | None | None | Excellent | Bad |

The results of Experiments 1 and 2 show that if the percentage content of the MN of the barrier layer 12 is set in a range equal to or more than 2 wt% and equal to or less than 22 wt%, a barrier property for sufficiently suppressing the transition of the plasticizer to the support can be secured in any of the non-elongation time and the 20% elongation time.

### (Experiment 3 Evaluation of Relationship between Degree of Saponification of PVA and Adhesiveness of Support -Barrier Layer: Evaluation of water resistance adhesion)

### (3-1 Preparation of Sample)

A support was prepared in the same manner as Experiment 1, and a degree of saponification of PVA to be used for a barrier layer was four stages of 80%, 90%, 95.5% and 98.5% (complete saponification). A barrier coating material was prepared by mixing the PVA of each degree of saponification and the MN, and was applied to form a barrier layer with the same amount and method as Experiment 1, and a sample 120 of the support film was prepared. The percentage content of the MN of the barrier layer was 10 wt%.

### (3-2 Experiment Procedure)

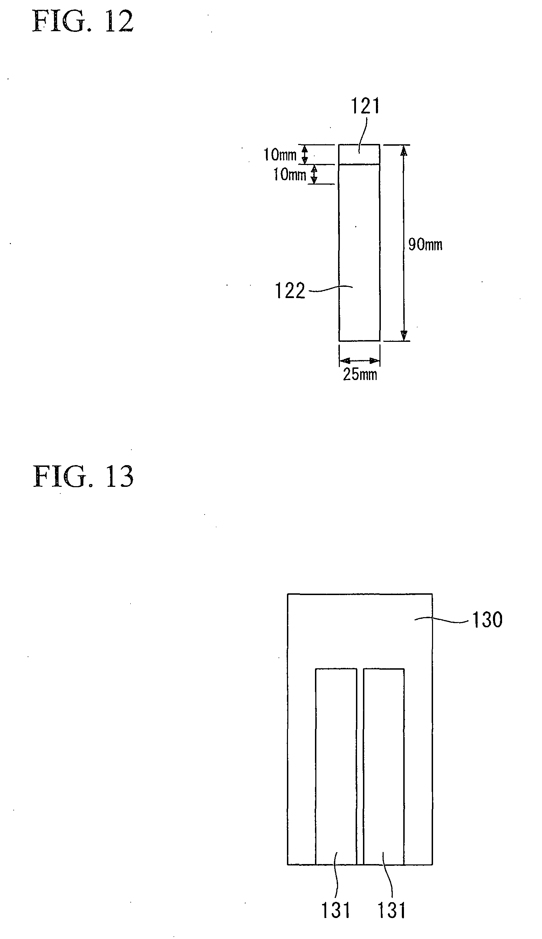

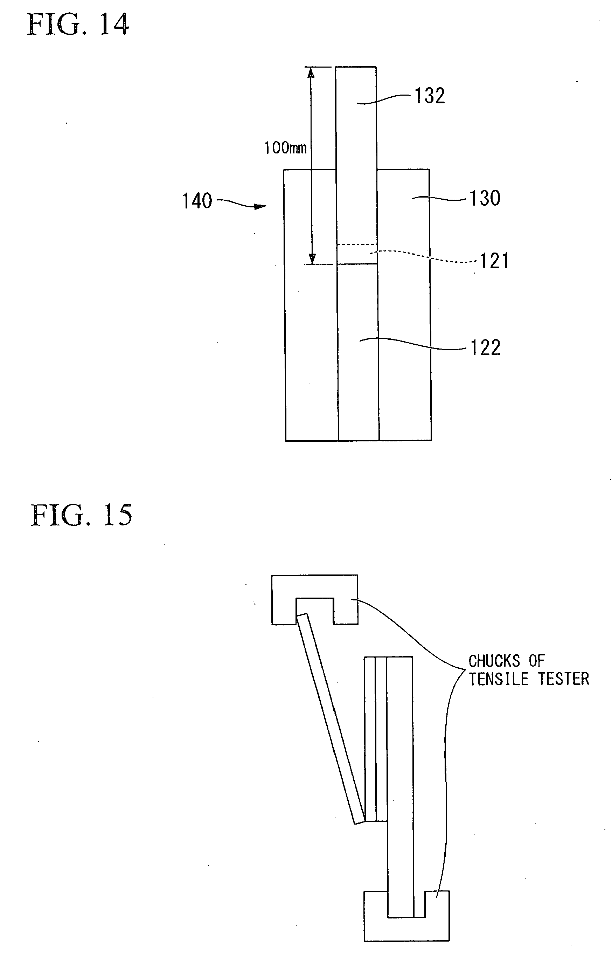

a. After cutting an adhesive tape 122 to be 30 mm × 100 mm and attaching a PET sheet 121 on which silicon was applied to one end in the longitudinal direction, the adhesive tape was attached to the barrier layer of the sample 120 as shown in FIG. 11.
b. The adhesive tape 122, the PET sheet 121 and the sample 120were cut to have a size of 25 mm × 90 mm as shown in FIG. 12.
c. As shown in FIG. 13, two double-sided tapes 131 having a size of 25 mm × 90 mm were attached to be in parallel to each other to an acrylic plate 130, and the support side of the cut adhesive tape 122 and the double-sided tapes 131 were adhered so as to cover two double-sided tapes 131. A part of double-sided tapes 131 which protruded in a width direction of the adhesive tape 122 was cut off to remove from the acrylic plate 130.
d. A reinforcement tape 132 having a size of 50 mm × 100 mm was prepared, and as shown in FIG. 14, the reinforcement tape 132 was attached to the end of the adhesive tape 122 which was not adhered to the sample 120 so as to interpose the PET sheet 121 in the thickness direction, to prepare an evaluation piece 140.
e. The evaluation piece 140 was dipped in water at 40°C and left for 30 minutes. At that time, the entire adhesive tape 122 was positioned in the water.
f. The evaluation piece 140 was picked up from the water after 30 minutes had passed, and was set in the tensile tester after wiping off the moisture. At that time, as shown in FIG. 15, the acrylic plate 130 was fixed to one chuck, and an end of a side which was not adhered to the PET sheet of the reinforcement tape 132 was fixed to another chuck.
g. The evaluation piece was pulled with a tension rate of 300 mm/min, and the measurement ended at the point of complete peel-off of the adhesive tape 122 from the support. An average value of tension values N of the tensile tester with a range of tension amount from 10 mm to 30 mm was set as a water resistance adhesion. Three evaluation pieces were prepared for a sample and the water resistance adhesion was evaluated.

### (3-3 Experiment Results)

The result is shown in Table 3. With the evaluation piece having the degree of saponification of PVA of equal to or less than 95.5%, the average value of the tension values N was equal to or more than 10 Newtons (N) and excellent water resistance adhesion was shown. With the evaluation piece having the degree of saponification of 98.5%, the water resistance adhesion was significantly degraded. Accordingly, in order to obtain excellent adhesiveness of the support and the barrier layer, it was considered that the degree of saponification of the water-soluble polymer was preferable to be equal to or less than 95.5%.

**[Table 3]**

| Degree of saponification ofPVA | Amount of MN (wt%) | Water resistance adhesion [N / 25 mm width] | | |
|---|---|---|---|---|
| | | n = 1 | n = 2 | n = 3 |
| 80 | 10 | 13.5 | 13.1 | 17.2 |
| 90 | | 10.6 | 10.2 | 10.6 |
| 95.5 | | 14.1 | 13 | 13.5 |
| 98.5 | | 1.9 | 2.6 | 2.1 |

As described above, in the support film of the present invention, by setting the percentage content of MN of the barrier layer to be equal to or more than 2 wt% and equal to or less than 22 wt%, it is possible to suitably maintain the barrier property even with elongation to an elongation rate of 20%. As a result, in any case of the non-elongation time and the elongation to an elongation rate of 20%, it is possible to suitably maintain a barrier property and to form a tape which suitably prevents transition of plasticizers of an adhesive layer to a support.

In addition, by setting a degree of saponification of PVA of the barrier layer to be equal to or more than 70% and equal to or less than 95.5%, it is possible to obtain excellent adhesiveness of the support and the barrier layer, and to configure a support film and a tape which can resist under more varied use conditions.

In addition, as shown in the following Table 4, when the percentage content of MN of the barrier layer was fixed to 10 wt% and several patterns of the degree of saponification of PVA were reviewed, it was determined that there was no significant effect to the barrier property in a range of the degree of saponification equal to or more than 90%.

**[Table 4]**

| Sample configuration | | | Evaluation | | | | | | | | | | | | | | | | | | | |
|---|---|---|---|---|---|---|---|---|---|---|---|---|---|---|---|---|---|---|---|---|---|---|
| Support | Barrier layer formula | | IPM | | | | | TA | | | | | MGIS | | | | | SMO | | | | |
| | Degree of saponification of PVA | Amount of MN | Elongation rate (%) | | | | | Elongation rate (%) | | | | | Elongation rate (%) | | | | | Elongation rate (%) | | | | |
| | % | wt% | 0 | 5 | 10 | 20 | 30 | 0 | 5 | 10 | 20 | 30 | 0 | 5 | 10 | 20 | 30 | 0 | 5 | 10 | 20 | 30 |
| Ethers 20 µm | 90 | 10 | Good | Good | Good | Good | Good | Good | Good | Good | Good | Good | Good | Good | Good | Good | Good | Bad | Bad | Bad | Bad | Bad |
| | 95.5 | 10 | Good | Good | Good | Good | Good | Good | Good | Good | Good | Good | Good | Good | Good | Good | Good | Bad | Bad | Bad | Bad | Bad |
| | 98.5 | 10 | Good | Good | Good | Good | Good | Good | Good | Good | Good | Good | Good | Good | Good | Good | Good | Good | Good | Bad | Bad | Bad |

As described above, the embodiment of the present invention has been described; however, the technique range of the present invention is not limited to the embodiment described above, and it is possible to change combinations of constituent elements of each embodiment, to add and remove various modifications to and from each constituent element in a range not departing from the purpose of the present invention.

For example, in the support film of the present invention, it is not essential to set a percentage content of a layered inorganic compound to the range described above. Accordingly, when the support film or the tape is used almost with no elongation, for example, a percentage content of a layered inorganic compound of the barrier layer may be beyond the range described above.

The present invention can be widely used for a tape for various purposes such as for medicine, for industrial use and the like.

## Claims

1. A support film for tape which is used for a tape, comprising:
a film-shaped support formed of polyurethane; and
a barrier layer which includes polyvinyl alcohol and a layered inorganic compound, and which is formed on one surface of the support,
wherein a degree of saponification of the polyvinyl alcohol is equal to or more than 70% and equal to or less than 95.5%, and wherein the film-shaped support can be elongated by a predetermined maximum elongation rate increasing equal to or more than 10% dimensionally.

2. The support film for tape according to Claim 1, wherein the layered inorganic compound is montmorillonite.

3. A tape comprising:
the support film for tape according to Claim 1 or 2; and
an adhesive layer which is formed on the barrier layer opposite to the support.

## Patentansprüche

1. Trägerfilm für ein Band, welches als Band verwendet wird, umfassend:
ein aus Polyurethan gebildeter, Film-förmiger Träger; und
eine Barriereschicht, die Polyvinylalkohol und eine schichtartige anorganische Verbindung einschließt und welche auf einer Oberfläche des Trägers gebildet ist,
wobei der Verseifungsgrad des Polyvinylalkohols gleich oder mehr als 70% und gleich oder weniger als 95,5% beträgt und wobei der Film-förmige Träger gestreckt werden kann durch ein vorbestimmtes maximales Streckausmaß, das eine Dimensionszunahme von gleich oder mehr als 10% ausmacht.

2. Trägerfilm für ein Band gemäß Anspruch 1, wobei die schichtartige anorganische Verbindung Montmorillonit ist.

3. Band aufweisend:
den Trägerfilm für ein Band gemäß Anspruch 1 oder 2, und
eine Klebeschicht, die auf der dem Träger entgegengesetzten Seite der Barriereschicht gebildet ist.

## Revendications

1. Film de support pour ruban qui est utilisé pour un ruban, comprenant :
un support en forme de film à base de polyuréthane ; et
une couche barrière qui comporte de l'alcool de polyvinyle et un composé inorganique agencé en couche, et qui est formée sur une surface du support,
dans lequel un degré de saponification de l'alcool de polyvinyle est supérieur ou égal à 70 % et inférieur ou égal à 95,5 %, et dans lequel le support en forme de film peut être allongé avec une augmentation de taux d'allongement maximum prédéterminée dimensionnellement supérieure ou égale à 10%.

2. Film de support pour ruban selon la revendication 1, dans lequel le composé inorganique agencé en couche est de la montmorillonite.

3. Ruban comprenant :
le film de support pour ruban selon la revendication 1 ou 2 ; et
une couche adhésive qui est formée sur la couche barrière opposée au support.
